(19) Europäisches Patentamt
European Patent Office
Office européen des brevets

(11) Veröffentlichungsnummer: **0 031 296**
**B1**

(12) **EUROPÄISCHE PATENTSCHRIFT**

(45) Veröffentlichungstag der Patentschrift: **07.08.85**

(21) Anmeldenummer: **80810370.9**

(22) Anmeldetag: **01.12.80**

(51) Int. Cl.⁴: **C 07 D 235/20,**
C 07 D 263/56,
C 07 D 405/04,
C 07 D 405/14,
C 07 D 409/14,
C 07 D 413/04,
C 07 D 413/06, C 07 D 413/14

# (54) Verfahren zur Herstellung von Benzazolylverbindungen.

(30) Priorität: **06.12.79 CH 10829/79**

(43) Veröffentlichungstag der Anmeldung:
**01.07.81 Patentblatt 81/26**

(45) Bekanntmachung des Hinweises auf die Patenterteilung:
**07.08.85 Patentblatt 85/32**

(84) Benannte Vertragsstaaten:
**CH DE FR GB IT LI**

(56) Entgegenhaltungen:
**DD-A- 94 996**
**DD-A- 97 209**
**DE-A-1 469 207**
**DE-A-2 306 050**
**DE-A-2 349 803**
**DE-A-2 436 279**
**DE-A-2 645 301**
**DE-A-2 715 567**
**DE-A-2 750 947**
**DE-A-2 809 117**

(73) Patentinhaber: **CIBA-GEIGY AG**
**Postfach**
**CH-4002 Basel (CH)**

(72) Erfinder: **Schreiber, Werner**
**Riehenstrasse 264**
**CH-4058 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 031 296 B1**

**Beschreibung**

Die vorliegende Erfindung betrifft ein Verfahren zur Herstellung von Benzazolylverbindungen.

Es sind aus der Literatur Verfahren zur Herstellung von Di-benzazolylverbindungen durch Kondensation von organischen Dicarbonsäuren mit Aminobenzolen in Gegenwart von organischen Lösungsmitteln und in Anwesenheit von Katalysatoren bekannt. So ist z.B. aus den US—PS 2 842 545 und 2 995 605 und der GB—PS 835 894 die Herstellung von α,β-Di-[aryloxazol-(2)-yl]-äthylenen aus 1-Amino-2-hydroxybenzol und Weinsäure oder Aepfelsäure in höhersiedenden Kohlenwasserstoffen der Benzolreihe bzw. chlorierten Benzolderivaten, wie Xylol oder Chlorbenzol bekannt. In der US—PS 3 103 518 wird die Herstellung von 2,5-Di-[benzimidazol-(2')-yl]-furanen durch Kondensation der Furan-2,5-dicarbonsäure mit einem 1,2-Diaminobenzol in Gegenwart von Borsäure und in Abwesenheit eines Lösungsmittels beschrieben, wobei darauf hingewiesen wird, dass die Anwesenheit eines hochsiedenden Lösungsmittels zu weniger guten Ausbeuten führt. Aehnliche 2,5-Di-[benzimidazol-(2')-yl]-furane werden gemäss der GB—PS 950 890 dadurch hergestellt, dass die Kondensation der Furan-2,5-dicarbonsäure mit dem 1,2-Diaminobenzol in Gegenwart einer hochsiedenden aliphatischen, gegebenenfalls verätherten Hydroxyverbindung, insbesondere Glycerin, als Lösungsmittel und Borsäure als wasserabspaltendes Mittel vorgenommen wird. In der US—PS 3 135 762 ist weiter die Herstellung von Bis(benzoxazolyl)-thiophenen aus o-Hydroxyamino-benzolen und Dicarbonsäuren in Gegenwart eines Katalysators, wie Zinkchlorid oder Borsäure und gegebenenfalls in einem höhersiedenden polaren Lösungsmittel beschrieben. Alle diese Verfahren sind aber mit dem Nachteil behaftet, dass die Endprodukte in unbefriedigenden Ausbeuten erhalten werden.

Der vorliegenden Erfindung liegt die Aufgabe zugrunde, ein Verfahren zu beschaffen, welches ermöglicht, Benzazolylverbindungen in besseren Ausbeuten und in kürzeren Reaktionszeiten herzustellen.

Es wurde überraschenderweise gefunden, dass Benzazolylverbindungen in guten Ausbeuten und in kürzeren Reaktionszeiten hergestellt werden können, wenn man die Kondensation der organischen Dicarbonsäure mit dem Aminobenzol in einem spezifischen Lösungsmittelgemisch durchführt.

Das erfindungsgemässe Verfahren zur Herstellung von Benzazolylverbindungen der Formel

(1) 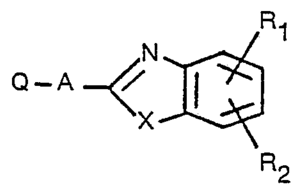

durch Kondensation, in Gegenwart eines Lösungsmittels, von Katalysatoren und bei erhöhter Temperatur, von organischen Carbonsäuren der Formeln

(2) HOOC—B und

(3) $HOOC—B_1$

worin B den 5-Carboxy-fur-(2)-yl-, $HOOC—CHOH—CH_2$—, HOOC—CHOH—CHOH— oder einen Benzofur-(2)-yl-rest und $B_1$ den 5-Carboxy-then(2)-yl-, $HOOC—CHOH—CH_2$— oder HOOC—CHOH—CHOH-rest bedeuten, mit Aminobenzolen der Formeln

(4) 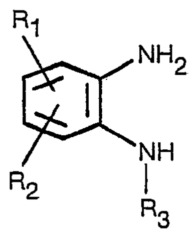 bzw. (5) 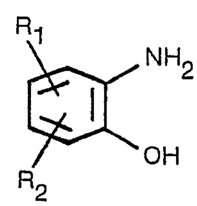

worin $R_1$ Wasserstoff, Halogen, $C_{1-4}$-alkyl, $C_{1-4}$-alkoxy, Hydroxy-$C_{1-4}$-alkyl, —COCN oder —COO—$C_{1-4}$-alkyl, $R_2$ Wasserstoff, Halogen, $C_{1-4}$-alkyl, $C_{1-4}$-alkoxy, Hydroxy-$C_{1-4}$-alkyl, —COCN oder —COO—$C_{1-4}$-alkyl, X Sauerstoff oder eine $R_3N$-Gruppe, worin $R_3$ für Wasserstoff, $C_{1-4}$-alkyl, Hydroxy-$C_{1-4}$-alkyl, Phenyl oder Benzyl steht, A 2,5-Furylen, 2,5-Thenylen, ein Brückenglied mit mindestens einer Doppelbindung zwischen 2 C-Atomen oder die direkte Bindung und Q einen Rest der Formel

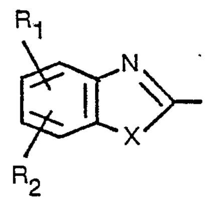

worin X, $R_1$ und $R_2$ die oben angegebene Bedeutung haben, und wenn A die direkte Bindung bedeutet, auch einen Rest der Formel

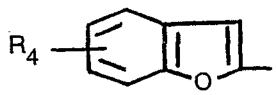

worin $R_4$ für Wasserstoff, $C_{1-4}$-alkyl, $C_{1-4}$-alkoxy oder Halogen steht, bedeuten, ist dadurch gekennzeichnet, dass man als Lösungsmittel das eutektische Gemisch von 73,5 Vol.-% Diphenyläther und 26,5 Vol.-% Diphenyl verwendet.

Als organische Carbonsäuren kommen für das erfindungsgemässe Verfahren z.B. Furan-2,5-dicarbonsäure, Thiophen-2,5-dicarbonsäure, Weinsäure, Aepfelsäure und Benzofuran-2-carbonsäure in Betracht.

Als Aminobenzole sind für das vorliegende Verfahren z.B. o-Diaminobenzole, deren eine Aminogruppe primär und deren andere Aminogruppe höchstens sekundär ist, sowie o-Aminophenole verwendbar, wie 4-Methyl-1,2-diamino-benzol, 4-Methoxy-1,2-diamino-benzol, 4,5-Dimethyl-1,2-diamino-benzol, 4-Isopropyl-1,2-diamino-benzol, 1-Amino-2-(methylamino)-benzol, o-Phenylendiamin, 1-Amino-2-(β-hydroxyäthl-amino)-benzol, 1-Amino-2-(äthylamino)-benzol, 1-Amino-2-(benzylamino)-benzol, 4-Chlor-1,2-diamino-benzol, 4-tert.-Butyl-1,2-diamino-benzol, 2-Aminophenol, 2-Amino-4-methylphenol, 2-Amino-5-methyl-phenol, 2-Amino-4,6-dimethylphenol, 2-Amino-4-tert.-butylphenol, 2-Amino-4-methoxyphenol, 2-Amino-4-chlorphenol und 2-Amino-4,6-dichlorphenol.

Als Katalysatoren kommen z.B. Zinkchlorid, Polyphosphorsäure, p-Toluolsulfonsäure und vorzugsweise Borsäure in Betracht.

Das erfindungsgemässe Verfahren eignet sich sehr gut zur Herstellung von Benzazolylverbindungen der Formel

(6) 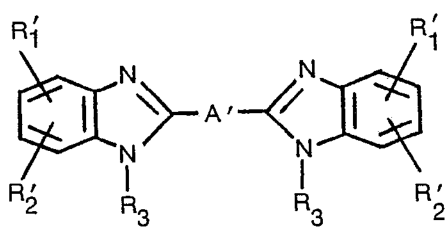

worin $R_1'$ Wasserstoff, $C_{1-4}$-alkyl oder Hydroxy-$C_{1-4}$-alkyl, $R_2'$ Wasserstoff, $C_{1-4}$-alkyl oder Hydroxy-$C_{1-4}$-alkyl, $R_3$ Wasserstoff, $C_{1-4}$-alkyl, Hydroxy-$C_{1-4}$-alkyl, Phenyl oder Benzyl und A' den 2,5-Furylen- oder den vinylenrest bedeuten, durch Kondensation einer Dicarbonsäure der Formel

(7) HOOC—B'—COOH

worin B' den 2,5-Furylen- oder den $—CH_2—CHOH$-rest bedeutet mit einem Aminobenzol der Formel

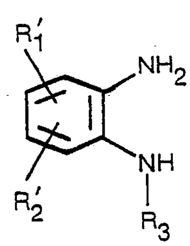

worin $R_1'$, $R_2'$ und $R_3$ die oben angegebene Bedeutung haben, sowie von Benzazolylverbindungen der Formel

(13) 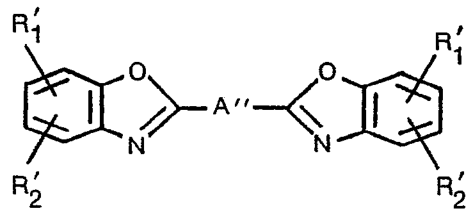

worin $R_1'$ Wasserstoff, $C_{1-4}$-alkyl oder Hydroxy-$C_{1-4}$-alkyl, $R_2'$ Wasserstoff, $C_{1-4}$-alkyl oder Hydroxy-$C_{1-4}$alkyl und A'' den 2,5-Thenylen- oder den Vinylenrest bedeuten, durch Kondensation einer Dicarbonsäure der Formel

(14) $HOOC—B_1'—COOH$

worin $B_1'$ den 2,5-Thenylen- oder den $—CH_2—CHOH$-rest bedeutet mit einem Aminobenzol der Formel

(15) 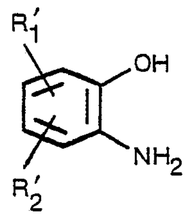

worin $R_1'$ und $R_2'$ die oben angegebene Bedeutung haben.

Das erfindungsgemässe Verfahren ist besonders interessant für die Herstellung von Benzazolylverbindungen der Formel

(9) 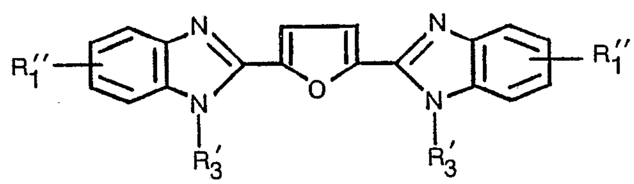

worin $R_1''$ Wasserstoff, Methyl oder β-Hydroxyäthyl und $R_3'$ Wasserstoff, Methyl oder Benzyl bedeuten, durch Kondensation von 2,5-Furandicarbonsäure mit einem Aminobenzol der Formel

(10) 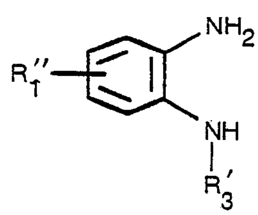

worin $R_1''$ und $R_3'$ die oben angegebene Bedeutung haben, und der Formel

(16) 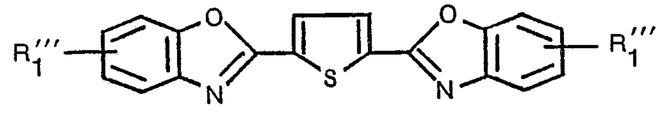

worin $R_1'''$ Wasserstoff, $C_{1-4}$-alkyl oder COO—$C_{1-4}$alkyl bedeutet, durch Kondensation von 2,5-Thiophendicarbonsäure mit einem Aminobenzol der Formel

(17) 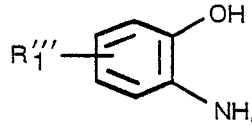

worin $R_1'''$ die oben angegebene Bedeutung hat.

Dem erfindungsgemässen Verfahren kommt eine besondere Bedeutung bei der Herstellung der Benzazolylverbindungen folgender Formeln zu:

(11) 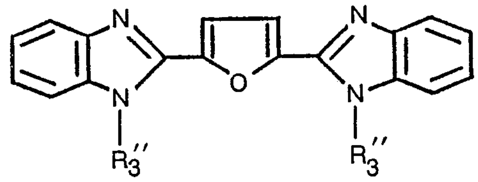

worin $R_3''$ Wasserstoff oder Methyl bedeutet, durch Kondensation von 2,5-Furandicarbonsäure mit einem Aminobenzol der Formel

(12) 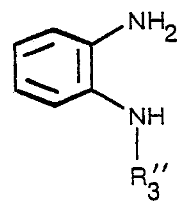

worin $R_3''$ die oben angegebene Bedeutung hat;

(18) 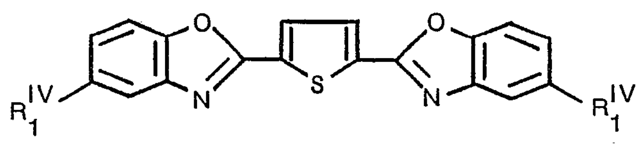

worin $R_1^{IV}$ Wasserstoff oder tert.-Butyl bedeutet, durch Kondensation von 2,5-Thiophendicarbonsäure mit einem Aminobenzol der Formel

(19) 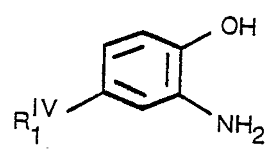

worin $R_1^{IV}$ die oben angegebene Bedeutung hat;

(20) 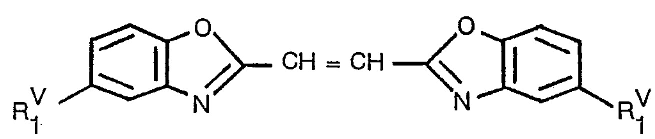

worin $R_1^{V}$ Wasserstoff oder Methyl bedeutet, durch Kondensation von Aepfelsäure der Formel

(21) $HOOC—CH_2—CHOH—COOH$

mit einem Aminobenzol der Formel

(22) 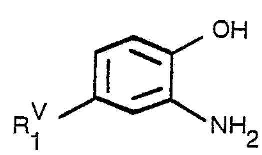

worin $R_1^{V}$ die oben angegebene Bedeutung hat;

(23) 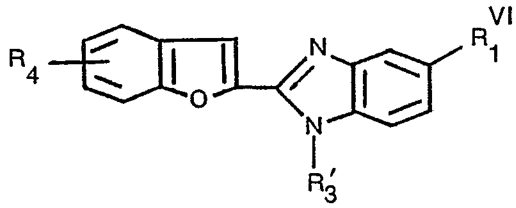

worin $R_1^{VI}$ Wasserstoff oder $C_{1-4}$-alkyl, $R_3'$ Wasserstoff, Methyl oder Benzyl und $R_4$ Wasserstoff, $C_{1-4}$-alkyl, $C_{1-4}$-alkoxy oder Halogen bedeuten, durch Kondensation einer Carbonsäure der Formel

(24) 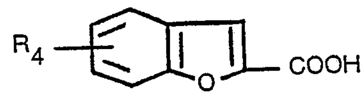

worin $R_4$ die oben angegebene Bedeutung hat, mit einem Aminobenzol der Formel

(25) 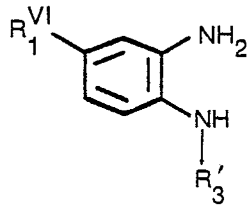

worin $R_1^{VI}$ und $R_3'$ die oben angegebene Bedeutung haben, und

(26) 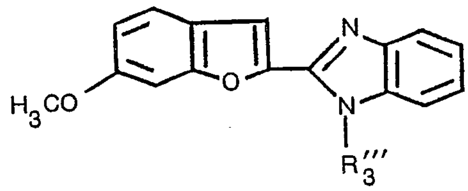

worin $R_3'''$ Methyl oder Benzyl bedeuten, durch Kondensation der 6-Methoxy-benzofuran-2-carbonsäure mit einem Aminobenzol der Formel

(27) 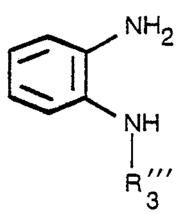

worin $R_3'''$ die oben angegebene Bedeutung haben.

Das vorliegende Verfahren eignet sich nicht nur zur Herstellung symmetrischer sondern auch zur Herstellung asymmetrischer Benzazolylverbindungen, welche durch Kondensation der organischen Carbonsäuren der Formeln (2) und (3) mit zwei verschiedenen Aminobenzolen der Formeln (4) und (5) hergestellt werden können. Zur Herstellung symmetrischer Benzazolylverbindungen setzt man die Carbonsäure mit dem Aminobenzol im Molecularverhältnis 1:2 um: für die asymmetrischen in einem solchen von 1:1:1 und für die Benzofuran-benzazole z.B. der Formel (23) in einem solchen von 1:1.

Die Kondensation der organischen Carbonsäuren der Formeln (2) und (3) mit den Aminobenzolen der Formeln (4) und (5) erfolgt bei Temperaturen zwischen 130° und 250°C unter Luftausschluss oder in Stickstoffatmosphäre und unter vermindertem Atmosphärendruck, z.B. 0,33 bis 10,6 Pa.

Zur Kondensation können die Carbonsäuren als solche oder in Form geeigneter funktioneller Derivate, z.B. in Form der Säurehalogenide verwendet werden.

Die nach dem vorliegenden Verfahren hergestellten Benzazolverbindungen sind bekannte Aufhellmittel für verschiedene organische Materialien.

Umsetzungen von organischen Dicarbonsäuren mit o-Diaminobenzolen oder o-Aminophenolen sind auch aus anderen Literaturstellen bekannt, so z.B. aus der DD—A—97209 die Umsetzung von 2-(4-Carboxyphenyl)-thiophen-5-carbonsäure mit o-Aminophenol in Abwesenheit eines Lösungsmittels, aus der DE—A—2715567 die Umsetzung von 4-[Benzoxazolyl-(2')]-naphthoesäure-(1) mit 1,2-Diaminobenzol in

Gegenwart eines Katalysators, aus der DE—A—2645301 die Umsetzung von Naphthalin-1,4-dicarbonsäure mit o-Aminophenolen oder o-Phenylendiaminen in einem Lösungsmittel und in An- oder Abwesenheit eines Katalysators aus der DE—A—23 06 050 die Umsetzung der Furan-2,5-dicarbonsäure mit 2-Hydroxy-4-phenoxy-anilinen in Gegenwert eines Katalysators und eines Lösungsmittels und aus der DE—A—1469207 die Umsetzung von Stilben-4,4'-dicarbonsäure mit o-Aminophenolen gegebenenfalls in Anwesenheit eines Katalysators und eines Lösungsmittels.

Die nachfolgenden Beispiele veranschaulichen die Erfindung ohne sie darauf zu begrenzen. Sofern nichts anderes vermerkt, bedeuten Prozente Gewichtsprozente.

## Beispiel 1

255,1 g 2-Amino-4-tert.-butyl-phenol (100%), 8,7 g Borsäure, 129,1 g Thiophen-2,5-dicarbonsäure (100%), 455 ml eines eutektischen Gemisches von 73,5 Vol.-% Diphenyläther und 26,5 Vol.-% Diphenyl werden in einem Reaktionsgefäss vorgelegt. Die Apparatur wird evakuiert und das Vakuum mit Stickstoff aufgehoben. Unter Rühren wird das Reaktionsgemisch auf 190—235°C erhitzt, wobei Wasser abdestilliert. Sobald 30 ml Wasser abdestilliert sind, wird die Apparatur auf ca. 10,6 Pa evakuiert und unter diesen Druckbedingungen 45 Minuten lang weiterkondensiert. Danach wird das Vakuum auf ca. 5,3 Pa erhöht und das Reaktionsgemisch 4 Stunden lang bei 230° bis 235°C gehalten. Durch Abdestillation von ca. 300 ml des eutektischen Gemisches Diphenyl/Diphenyläther im Vakuum wird das Reaktionsgemisch eingeengt, wobei sich dasselbe auf 140—150°C abkühlt. Alsdann hebt man das Vakuum mit Stickstoff auf und sobald das Produkt auskristallisiert ist, lässt man 560 ml Methanol langsam zufliessen. Das Ganze wird 30 Minuten lang am Rückfluss gekocht, abgekühlt und der kristalline Niederschlag abgenutscht, mit 500 ml Methanol und anschliessend 300 ml deionisiertem Wasser gewaschen, und im Vakuumschrank bei 100°C getrocknet. Man erhält 315 g (97% der Theorie) der Verbindung der Formel

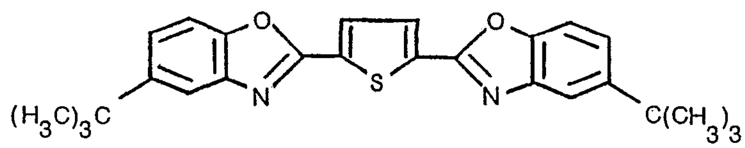

welche nach Umkristallisation aus dem eutektischen Gemisch einen Smp. von 199—200°C aufweist.

## Beispiel 2

Verfährt man wie im Beispiel 1 angegeben, verwendet aber anstelle von 255,2 g 2-Amino-4-tert.-butylphenol 168,6 g 2-Amino-phenol (100%), so erhält man 232 g (97% der Theorie) der Verbindung der Formel

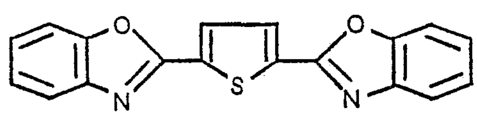

mit einem Smp. von 216—217°C.

## Beispiel 3

In einem Reaktionsgefäss werden 123,1 g 2-Amino-4-methyl-phenol, 67,7 g Aepfelsäure, 367,5 g (346,7 Vol.-%) Diphenyläther und 132,5 g (125 Vol.-%) Diphenyl vorgelegt. Das Reaktionsgemisch wird auf 1,19 Pa evakuiert und das Reaktionsgemisch auf 135°C erhitzt und 3 Stunden bei dieser Temperatur gehalten. Das Vakuum wird mit Stickstoff aufgehoben und es werden 3 g Borsäure zugegeben. Das Reaktionsgefäss wird erneut evakuiert und das Gemisch auf 155°C erhitzt. Das Ganze wird bei dieser Temperatur und einem Druck von ca. 0,66 Pa während 13 Stunden kondensiert. Danach wird das Reaktionsgemisch eingedampft und abgekühlt.

Das auskristallisierte Produkt wird nun mit 500 ml Methanol versetzt, 30 Minuten lang am Rückfluss erhitzt, darauf auf 20°C abkühlen gelassen und bei dieser Temperatur eine Stunde lang nachgerührt. Danach wird der Niederschlag abgenutscht, 4 mal mit je 100 ml Methanol und anschliessend mit 400 ml deionisiertem Wasser gewaschen. Man erhält 127 g (86% der Theorie) der Verbindung der Formel

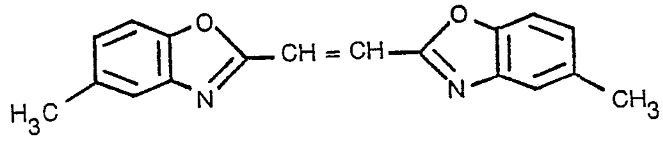

die nach Umkristallisation aus Xylol einen Smp. von 183—184°C hat.

Beispiel 4

Verfährt man analog wie im Beispiel 1 angegeben, verwendet aber anstelle der Thiophen-2,5-dicarbonsäure 117 g Furan-2,5-dicarbonsäure und anstelle von 2-Amino-4-tert.butylphenol 166,5 g Phenylendiamin oder 188,5 g N-Methyl-phenylendiamin, so erhält man die Verbindung der Formel

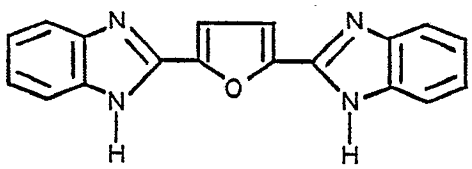

vom Smp. 310°C bzw. der Formel

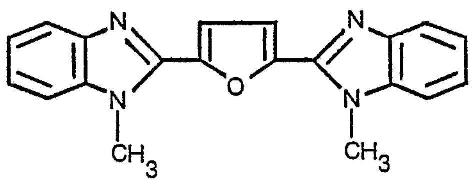

vom Smp. 272°C.

Beispiel 5

In einem Reaktionsgefäss werden 96,1 g 6-Methoxy-benzofuran-2-carbonsäure, 99,2 g N-Benzyl-o-phenylendiamin, 5 g Borsäure und 400 ml des eutektischen Gemisches Diphenyl/Diphenyläther vorgelegt. Die Suspension wird unter Stickstoffatmosphäre und 0,66 bis 1,99 Pa auf 170—180°C erwärmt und 6 Stunden lang in diesem Temperaturbereich gehalten. Die Reaktionslösung wird dann eingeengt und auf Raumtemperatur abgekühlt, wobei das Produkt auskristallisiert. Man erhält 147 g der Verbindung der Formel

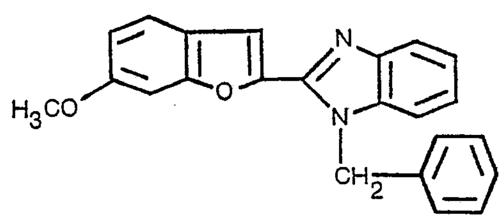

vom Smp. 142°C.

**Patentansprüche**

1. Verfahren zur Herstellung von Benzazolylverbindungen der Formel

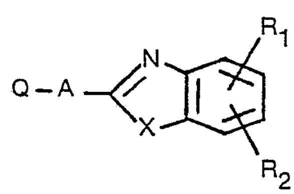

durch Kondensation, in Gegenwart eines Lösungsmittels von Katalysatoren und bei erhöhter Temperatur, von organischen Carbonsäuren der Formeln

HOOC—B und

$HOOC—B_1$

worin B den 5-Carboxy-fur-(2)-yl-, $HOOC—CHOH—CH_2—$, HOOC—CHOH—CHOH oder einen $R^4$-substituierten Benzofur-(2)-yl-rest und $B_1$ den 5-Carboxy-then-(2)-yl-, $HOOC—CHOH—CH_2—$ oder HOOC—CHOH—CHOH-rest bedeuten, mit Aminobenzolen der Formeln

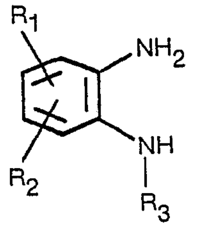 bzw. 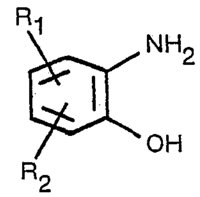

worin $R_1$ Wasserstoff, Halogen, $C_{1-4}$-alkyl, $C_{1-4}$-alkoxy, Hydroxy-$C_{1-4}$-alkyl, —COCN oder —COO—$C_{1-4}$-alkyl, $R_2$ Wasserstoff, Halogen, $C_{1-4}$-alkyl, $C_{1-4}$-alkoxy, Hydroxy-$C_{1-4}$-alkyl, —COCN oder —COO—$C_{1-4}$-alkyl, X Sauerstoff oder eine $R_3N$-Gruppe, worin $R_3$ für Wasserstoff, $C_{1-4}$-alkyl, Hydroxy-$C_{1-4}$-alkyl, Phenyl oder Benzyl steht, A 2,5-Furylen, 2,5-Thenylen, ein Brückenglied mit mindestens einer Doppelbindung zwischen 2 C-Atomen oder die direkte Bindung und Q einen Rest der Formel

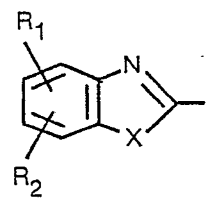

worin X, $R_1$ und $R_2$ die oben angegebene Bedeutung haben, und wenn A die direkte Bindung bedeutet, auch einen Rest der Formel

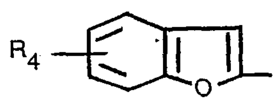

worin $R_4$ für Wasserstoff, $C_{1-4}$-alkyl, $C_{1-4}$-alkoxy oder Halogen steht, bedeuten, ist dadurch gekennzeichnet, dass man als Lösungsmittel das eutektische Gemisch von 73,5 Vol.-% Diphenyläther und 26,5 Vol.-% Diphenyl verwendet.

2. Verfahren gemäss Anspruch 1, worin die Kondensation bei reduziertem Druck vorgenommen wird.

3. Verfahren gemäss Anspruch 2 zur Herstellung von Benzazolylverbindungen der Formel

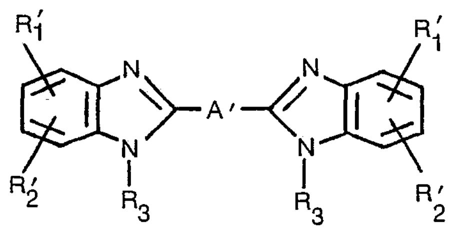

worin $R_1'$ Wasserstoff, $C_{1-4}$-alkyl oder Hydroxy-$C_{1-4}$-alkyl, $R_2'$ Wasserstoff, $C_{1-4}$-alkyl oder Hydroxy-$C_{1-4}$-alkyl, $R_3$ Wasserstoff, $C_{1-4}$-alkyl, Hydroxy-$C_{1-4}$-alkyl, Phenyl oder Benzyl und A' den 2,5-Furylen- oder den Vinylenrest bedeuten, durch Kondensation einer Dicarbonsäure der Formel

$$HOOC—B'—COOH$$

worin B' den 2,5-Furylen- oder den $—CH_2—CHOH$-rest bedeutet mit einem Aminobenzol der Formel

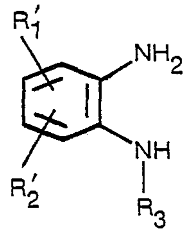

worin $R_1'$, $R_2'$ und $R_3$ die oben angegebene Bedeutung haben.

4. Verfahren gemäss Anspruch 3 zur Herstellung von Benzazolylverbindungen der Formel

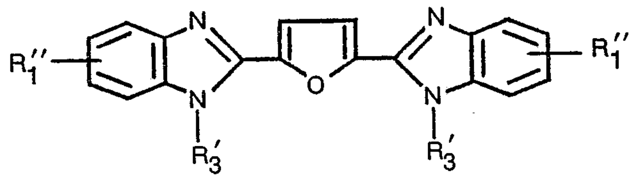

worin $R_1''$ Wasserstoff, Methyl oder β-Hydroxyäthyl und $R_3'$ Wasserstoff, Methyl oder Benzyl bedeuten, durch Kondensation von 2,5-Furandicarbonsäure mit einem Aminobenzol der Formel

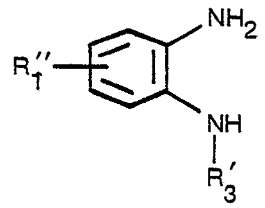

worin $R_1''$ und $R_3'$ die oben angegebene Bedeutung haben.

5. Verfahren gemäss Anspruch 4 zur Herstellung von Benzazolylverbindungen der Formel

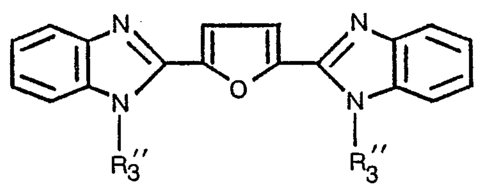

worin $R_3''$ Wasserstoff oder Methyl bedeutet, durch Kondensation von 2,5-Furandicarbonsäure mit einem Aminobenzol der Formel

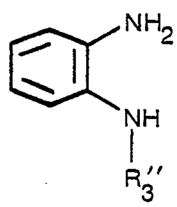

worin $R_3''$ die oben angegebene Bedeutung hat.

6. Verfahren gemäss Anspruch 2 zur Herstellung von Benzazolylverbindungen der Formel

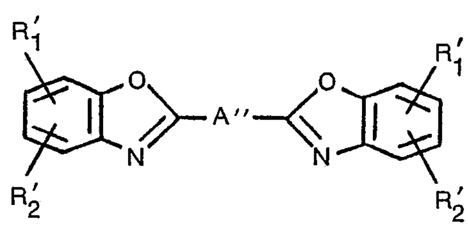

worin $R_1'$ Wasserstoff, $C_{1-4}$-alkyl oder Hydroxy-$C_{1-4}$-alkyl, $R_2'$ Wasserstoff, $C_{1-4}$-alkyl oder Hydroxy-$C_{1-4}$-alkyl und A'' den 2,5-Thenylen oder den Vinylenrest bedeuten, durch Kondensation einer Dicarbonsäure der Formel

$$HOOC—B_1'—COOH$$

worin $B_1'$ den 2,5-Thenylen- oder den $—CH_2—CHOH$-rest bedeutet mit einem Aminobenzol der Formel

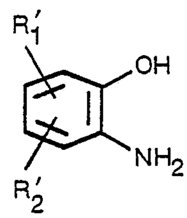

worin $R_1'$ und $R_2'$ die oben angegebene Bedeutung haben.

7. Verfahren gemäss Anspruch 2 zur Herstellung von Benzazolylverbindungen der formel

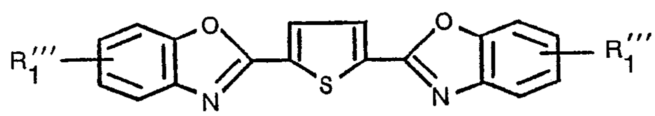

worin $R_1'''$ Wasserstoff, $C_{1-4}$-alkyl oder COO—$C_{1-4}$-alkyl bedeutet, durch Kondensation von 2,5-Thiophendicarbonsäure mit einem Aminobenzol der Formel

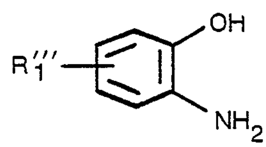

worin $R_1'''$ die oben angegebene Bedeutung hat.

8. Verfahren gemäss Anspruch 7 zur Herstellung von Benzazolylverbindungen der Formel

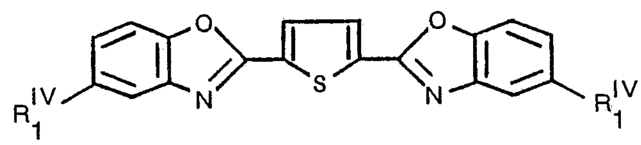

worin $R_1^{IV}$ Wasserstoff oder tert.-Butyl bedeutet, durch Kondensation von 2,5-Thiophendicarbonsäure mit einem Aminobenzol der Formel

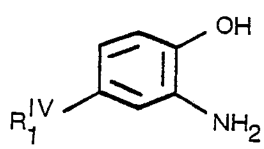

worin $R_1^{IV}$ die oben angegebene Bedeutung hat.

9. Verfahren gemäss Anspruch 6 zur Herstellung von Benzazolylverbindungen der Formel

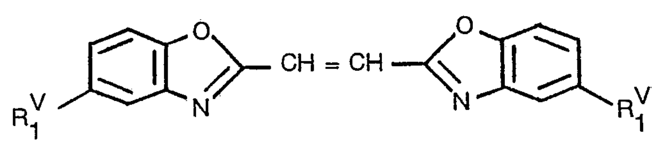

worin $R_1^{V}$ Wasserstoff oder Methyl bedeutet, durch Kondensation von Aepfelsäure der Formel

$$HOOC—CH_2—CHOH—COOH$$

mit einem Aminobenzol der Formel

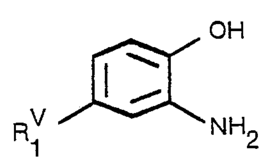

worin $R_1^V$ die oben angegebene Bedeutung hat.

10. Verfahren gemäss Anspruch 1 zur Herstellung von Benzazolylverbindungen der Formel

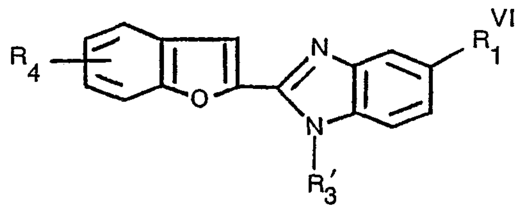

worin $R_1^{VI}$ Wasserstoff oder $C_{1-4}$-alkyl, $R_3'$ Wasserstoff, Methyl oder Benzyl und $R_4$ Wasserstoff, $C_{1-4}$-alkyl, $C_{1-4}$-alkoxy oder Halogen bedeuten, durch Kondensation einer Carbonsäure der Formel

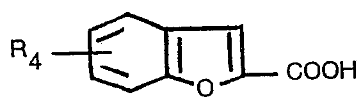

worin $R_4$ die oben angegebene Bedeutung hat, mit einem Aminobenzol der Formel

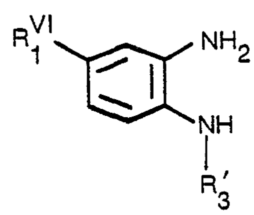

worin $R_1^{VI}$ und $R_3'$ die oben angegebene Bedeutung haben.

11. Verfahren gemäss Anspruch 10 zur Herstellung von Benzazolylverbindungen der Formel

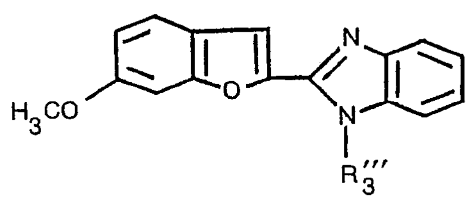

worin $R_3'''$ Methyl oder Benzyl bedeutet, durch Kondensation der 6-Methoxybenzofuran-2-carbonsäure mit einem Aminobenzol der Formel

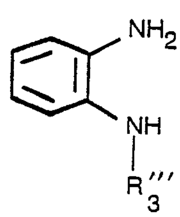

worin $R_3'''$ die oben angegebene Bedeutung hat.

## Revendications

1. Procédé pour la préparation de composés benzazolyliques de formule

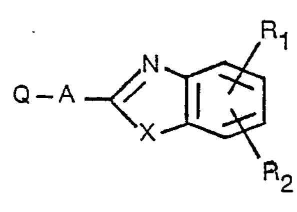

par condensation, en présence d'un solvant, de catalyseurs et à haute température, d'acides carboxyliques organiques de formules

HOOC—B et

$HOOC—B_1$

où B signifie les radicaux 5-carboxy-fur-(2)-yle-, $HOOC—CHOH—CH_2—$, HOOC—CHOH—CHOH— ou un radical benzofur-(2)-yle substitué par $R^4$ et $B_1$, le radical 5-carboxy-thén-(2)-yle, $HOOC—CHOH—CH_2—$ ou HOOC—CHOH—CHOH—, avec des aminobenzènes de formules

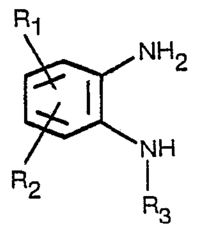

ou

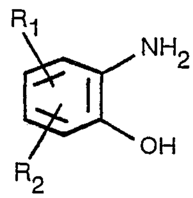

où $R_1$ est l'hydrogène, un halogène, un radical alkyle en $C_{1-4}$, alcoxy en $C_{1-4}$, hydroxy-alkyle en $C_{1-4}$, —COCN ou —COO-alkyle en $C_{1-4}$; $R_2$ est l'hydrogène, un halogène, un radical alkyle en $C_{1-4}$, alcoxy en $C_{1-4}$, hydroxy-alkyle en $C_{1-4}$, —COCN ou —COO-alkyle en $C_{1-4}$; X est l'oxygène ou un groupe $R_3N$, où $R_3$ représente l'hydrogène, un radical alkyle en $C_{1-4}$, hydroxy-alkyle en $C_{1-4}$, phényle ou benzyle; A est le radical 2,5-furylène, 2,5-thénylène, un élément de pontage avec au moins une double liaison entre deux atomes de C ou la liaison directe, et Q est un radical de formule

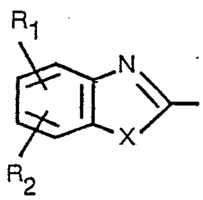

où X, $R_1$ et $R_2$ ont la signification donnée ci-dessus, et aussi, quand A est la liaison directe, un radical de formule

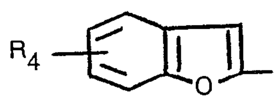

où $R_4$ représente l'hydrogène, un radical alkyle en $C_{1-4}$, alcoxy en $C_{1-4}$ ou un halogène, caractérisé en ce qu'on utilise en tant que solvant le mélange eutectique de 73,5 %en volume de diphényléther et de 26,5 % en volume de diphényle.

2. Procédé selon la revendication 1, dans lequel la condensation est réalisée sous pression réduite.

3. Procédé selon la revendication 2 pour la préparation de composés benzazolyliques de formule

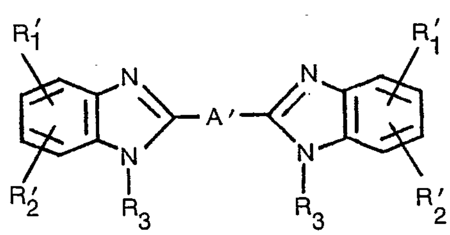

où $R_1'$ est l'hydrogène, un radical alkyle en $C_{1-4}$ ou hydroxy-alkyle en $C_{1-4}$, $R_2'$ est l'hydrogène, un radical alkyle en $C_{1-4}$ ou hydroxy-alkyle en $C_{1-4}$; $R_3$ est l'hydrogène, un radical alkyle en $C_{1-4}$, hydroxy-alkyle en $C_{1-4}$, phényle ou benzyle, et A' est le radical 2,5-furylène ou le radical vinylène, par condensation d'un acide dicarboxylique de formule

HOOC—B'—COOH

où B' est le radical 2,5-furylène ou le radical $—CH_2—CHOH$, avec un aminobenzène de formule

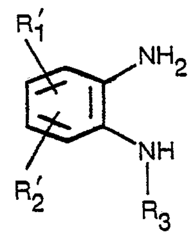

où $R_1'$ $R_2'$ et $R_3$ ont la signification donnée ci-dessus.

4. Procédé selon la revendication 3 pour la préparation de composés benzazolyliques de formule

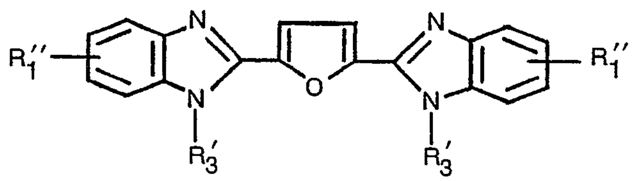

où $R_1''$ est l'hydrogène, le radical méthyle ou β-hydroxyéthyle, et $R_3'$ est l'hydrogène, le radical méthyle ou benzyle, par condensation d'acide 2,5-furannedicarboxylique avec un aminobenzène de formule

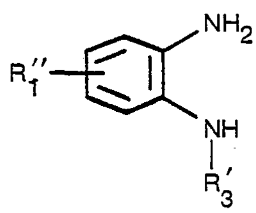

où $R_1''$ et $R_3'$ ont la signification donnée ci-dessus.

5. Procédé selon la revendication 4, pour la préparation de composés benzazolyliques de formule

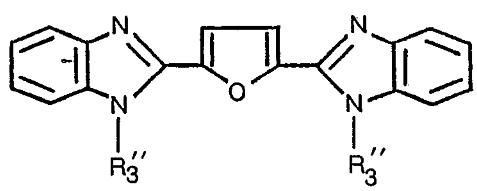

où $R_3''$ est l'hydrogène ou le radical méthyle, par condensation d'acide 2,5-furannedicarboxylique avec un aminobenzène de formule

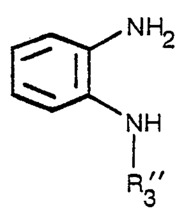

où $R_3''$ a la signification donnée ci-dessus.

6. Procédé selon la revendication 2, pour la préparation de composés benzazolyliques de formule

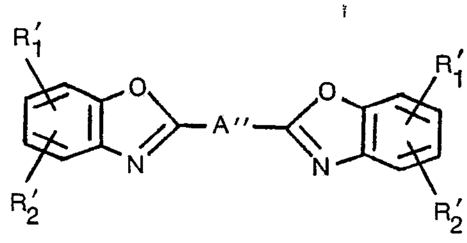

où $R_1'$ est l'hydrogène, un radical alkyle en $C_{1-4}$ ou hydroxy-alkyle en $C_{1-4}$; $R_2'$ est l'hydrogène, un radical

alkyle en $C_{1-4}$ ou hydroxy-alkyle en $C_{1-4}$, et A″ est le radical 2,5-thénylène ou le radical vinylène, par condensation d'un acide dicarboxylique de formule

$$HOOC—B_1'—COOH$$

où $B_1'$ représente le radical 2,5-thénylène ou le radical $—CH_2—CHOH$, avec un aminobenzène de formule

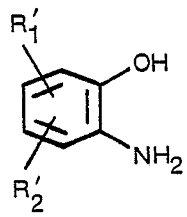

où $R_1'$ et $R_2'$ ont la signification donnée ci-dessus.

7. Procédé selon la revendication 2, pour la préparation de composés benzazolyliques de formule

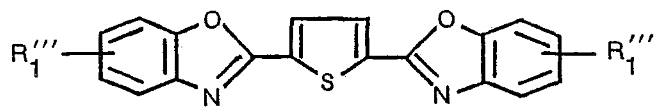

où $R_1'''$ est l'hydrogène, un radical alkyle en $C_{1-4}$ ou COO-alkyle en $C_{1-4}$, par condensation d'acide 2,5-thiophènedicarboxylique avec un aminobenzène de formule

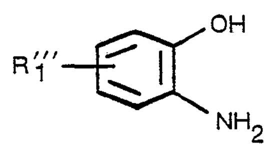

où $R_1'''$ a la signification donnée ci-dessus.

8. Procédé selon la revendication 7, pour la préparation de composés benzazolyliques, de formule

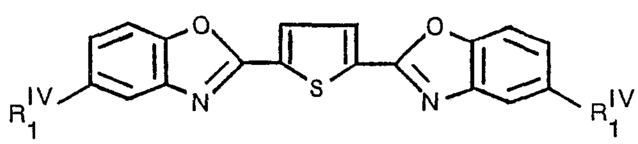

où $R_1^{IV}$ est l'hydrogène ou le radical tert-butyle, par condensation d'acide 2,5-thiophènedicarboxylique avec un aminobenzène de formule

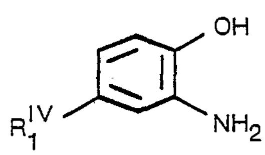

où $R_1^{IV}$ a la signification donnée ci-dessus.

9. Procédé selon la revendication 6, pour la préparation de composés benzazolyliques, de formule

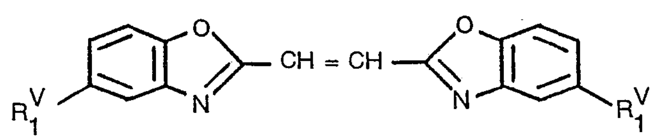

où $R_1^{V}$ est l'hydrogène ou le radical méthyle, par condensation d'acide malique de formule

$$HOOC—CH_2—CHOH—COOH$$

avec un aminobenzène de formule

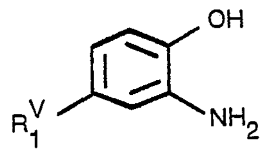

où $R_1^V$ a la signification donnée ci-dessus.

10. Procédé selon la revendication 1, pour la préparation de composés benzazolyliques de formule

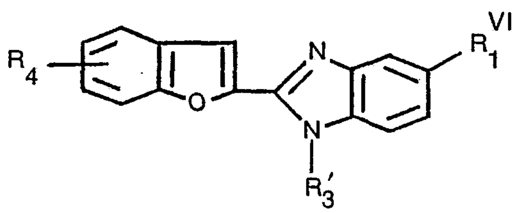

où $R_1^{VI}$ est l'hydrogène ou un radical alkyle en $C_{1-4}$; $R_3'$ est l'hydrogène, le radical méthyle ou benzyle, et $R_4$ est l'hydrogène, un radical alkyle en $C_{1-4}$, alcoxy en $C_{1-4}$ ou un halogène, par condensation d'un acide carboxylique de formule

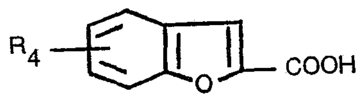

où $R_4$ a la signification donnée ci-dessus, avec un aminobenzène de formule

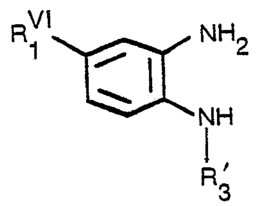

où $R_1^{VI}$ et $R_3'$ ont la signification donnée ci-dessus.

11. Procédé selon la revendication 10, pour la préparation de composés benzazolyliques de formule

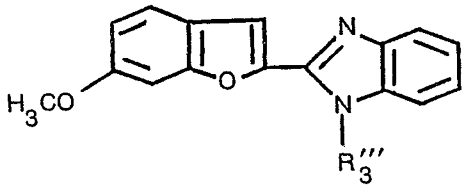

où $R_3'''$ est le radical méthyle ou benzyle, par condensation de l'acide 6-méthoxy-benzofuranne-2-carboxylique avec un aminobenzène de formule

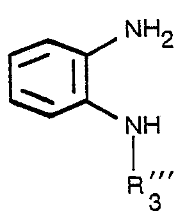

où $R_3'''$ a la signification donnée ci-dessus.

**Claims**

1. A process for the preparation of benzazolyl compounds of the formula

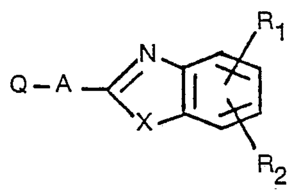

by condensation, in the presence of a solvent and a catalyst, and at elevated temperature, of organic carboxylic acids of the formulae

HOOC—B and

$HOOC—B_1$

wherein B is the 5-carboxy-fur-(2)-yl radical, the $HOOC—CHOH—CH_2—$ or HOOC—CHOH—CHOH— radical or an $R^4$-substituted benzoxazol-(2)-yl radical, and $B_1$ is the 5-carboxy-then-(2)-yl radical, $HOOC—CHOH—CH_2—$ or HOOC—CHOH—CHOH— radical, with aminobenzenes of the formulae

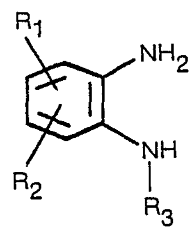

and

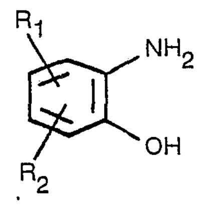

wherein $R_1$ is hydrogen, halogen, $C_{1-4}$alkyl, $C_{1-4}$alkoxy, $C_{1-4}$hydroxyalkyl, —COCN or —COO—$C_{1-4}$alkyl, $R_2$ is hydrogen, halogen, $C_{1-4}$alkyl, $C_{1-4}$alkoxy, $C_{1-4}$hydroxyalkyl, —COCN or —COO—$C_{1-4}$alkyl, X is oxygen or a $R_3N—$ group, wherein $R_3$ is hydrogen, $C_{1-4}$alkyl, $C_{1-4}$hydroxyalkyl, phenyl or benzyl, A is 2,5-furylene, 2,5-thenylene, a bridge member containing at least one double bond between 2 carbon atoms, or is the direct bond, and Q is a radical of the formula

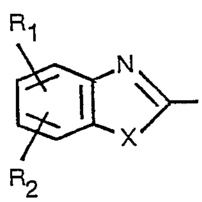

wherein X, $R_1$ and $R_2$ are as defined above, and, if A is the direct bond, Q is also a radical of the formula

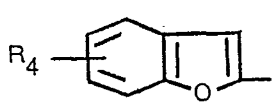

wherein $R_4$ is hydrogen, $C_{1-4}$alkyl, $C_{1-4}$alkoxy or halogen, which process comprises the use of a eutectic mixture of 73.5 vol % of diphenyl ether and 26.5 vol % of diphenyl as solvent.

2. A process according to claim 1, wherein the condensation is carried out under reduced pressure.

3. A process according to claim 2 for the preparation of benzazolyl compounds of the formula

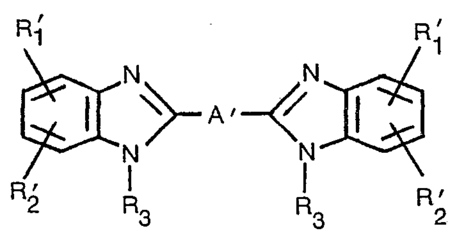

wherein $R_1'$ is hydrogen, $C_{1-4}$alkyl or $C_{1-4}$hydroxyalkyl, $R_2'$ is hydrogen, $C_{1-4}$alkyl or $C_{1-4}$hydroxyalkyl, $R_3$ is hydrogen, $C_{1-4}$alkyl, $C_{1-4}$hydroxyalkyl, phenyl or benzyl, and A' is the 2,5-furylene radical or the vinylene radical, which process comprises condensing a dicarboxylic acid of the formula

$$HOOC—B'—COOH$$

wherein B' is the 2,5-furylene radical or the $—CH_2—CHOH—$ radical, with an aminobenzene of the formula

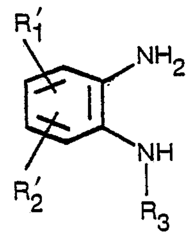

wherein $R_1'$, $R_2'$ and $R_3$ are as defined above.

4. A process according to claim 3 for the preparation of benzazolyl compounds of the formula

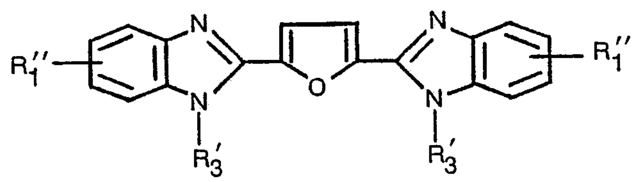

wherein $R_1''$ is hydrogen, methyl or β-hydroxyethyl, and $R_3'$ is hydrogen, methyl or benzyl, which process comprises condensing 2,5-furanedicarboxylic acid with an aminobenzene of the formula

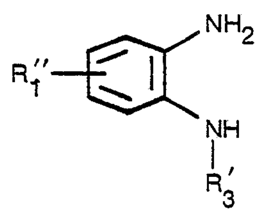

wherein $R_1''$ and $R_3'$ are as defined above.

5. A process according to claim 4 for the preparation of benzazolyl compounds of the formula

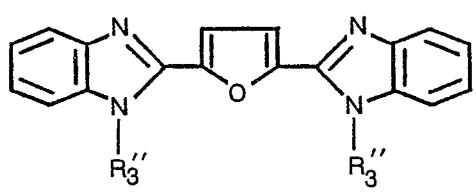

wherein $R_3''$ is hydrogen or methyl, which process comprises condensing 2,5-furanedicarboxylic acid with an aminobenzene of the formula

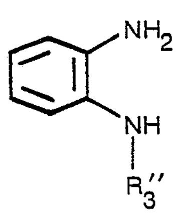

wherein $R_3''$ is as defined above.

6. A process according to claim 2 for the preparation of benzazolyl compounds of the formula

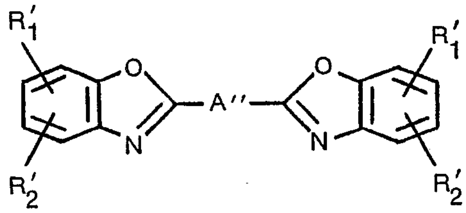

wherein $R_1'$ is hydrogen, $C_{1-4}$alkyl or $C_{1-4}$hydroxyalkyl, $R_2'$ is hydrogen, $C_{1-4}$alkyl or $C_{1-4}$hydroxyalkyl, and A'' is the 2,5-thenylene radical or the vinylene radical, which process comprises condensing a dicarboxylic acid of the formula

HOOC—$B_1'$—COOH

wherein $B_1'$ is the 2,5-thenylene radical or the —$CH_2$—CHOH— radical, with an aminobenzene of the formula

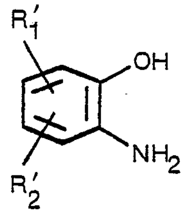

wherein $R_1'$ and $R_2'$ are as defined above.

7. A process according to claim 2 for the preparation of benzazolyl compounds of the formula

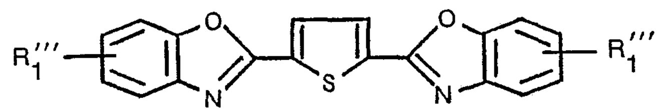

wherein $R_1'''$ is hydrogen, $C_{1-4}$alkyl or COO—$C_{1-4}$alkyl, which process comprises condensing 2,5-thiophenedicarboxylic acid with an aminobenzene of the formula

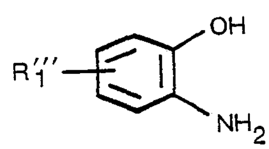

wherein $R_1'''$ is as defined above.

8. A process according to claim 7 for the preparation of benzazolyl compounds of the formula

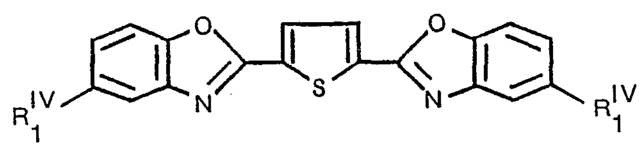

wherein $R_1^{IV}$ is hydrogen or tert-butyl, which process comprises condensing 2,5-thiophenedicarboxylic acid with an aminobenzene of the formula

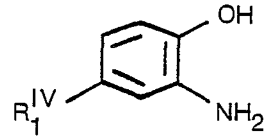

wherein $R_1^{IV}$ is as defined above.

9. A process according to claim 6 for the preparation of benzazolyl compounds of the formula

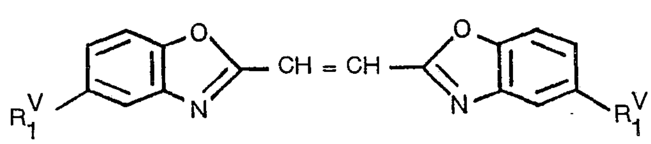

wherein $R_1^V$ is hydrogen or methyl, which process comprises condensing malic acid of the formula

$$HOOC—CH_2—CHOCH—COOH$$

with an aminobenzene of the formula

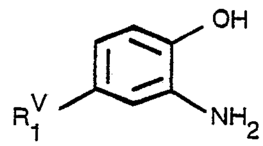

wherein $R_1^V$ is as defined above.

10. A process according to claim 1 for the preparation of benzazolyl compounds of the formula

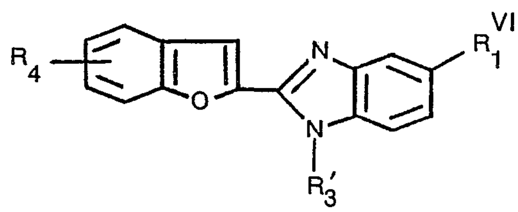

wherein $R_1^{VI}$ is hydrogen or $C_{1-4}$alkyl, $R_3'$ is hydrogen, methyl or benzyl, and $R_4$ is hydrogen, $C_{1-4}$alkyl, $C_{1-4}$alkoxy or halogen, which process comprises condensing a carboxylic acid of the formula

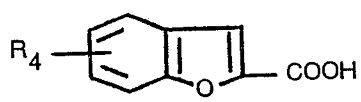

wherein $R_4$ is as defined above, with an aminobenzene of the formula

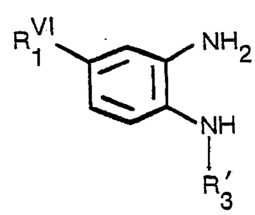

wherein $R_1^{VI}$ and $R_3'$ are as defined above.

11. A process according to claim 10 for the preparation of benzazolyl compounds of the formula

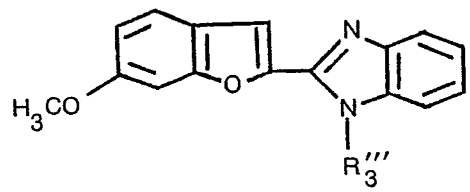

wherein $R_3'''$ is methyl or benzyl, which process comprises condensing 6-methoxybenzofurane-2-carboxylic acid with an aminobenzene of the formula

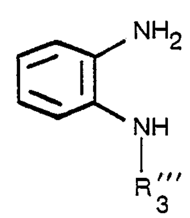

wherein $R_3'''$ is as defined above.